Europäisches Patentamt

European Patent Office

Office européen des brevets

(19)

(11) Veröffentlichungsnummer: **0 100 448**
**B1**

(12) # EUROPÄISCHE PATENTSCHRIFT

(45) Veröffentlichungstag der Patentschrift:
27.08.86

(51) Int. Cl.⁴: **A 01 N 25/04**, A 61 K 31/685

(21) Anmeldenummer: 83106464.7

(22) Anmeldetag: 02.07.83

(54) Flüssige Wirkstofformulierungen und ihre Verwendung zum Herstellen von Mikroemulsionen.

(30) Priorität: 09.07.82 DE 3225706

(43) Veröffentlichungstag der Anmeldung:
15.02.84 Patentblatt 84/7

(45) Bekanntmachung des Hinweises auf die Patenterteilung:
27.08.86 Patentblatt 86/35

(84) Benannte Vertragsstaaten:
AT BE CH DE FR GB IT LI LU NL SE

(56) Entgegenhaltungen:
EP - A - 0 082 437
DE - A - 3 010 041
DE - A - 3 042 365

(73) Patentinhaber: A. Nattermann & Cie. GmbH,
Nattermannallee 1, D-5000 Köln 30 (DE)

(72) Erfinder: Posanski, Ulrich, Dr., Husslerlstrasse 8,
D-7800 Freiburg (DE)
Erfinder: Ghyczy, Miklos, Dr. Dipl.-Chem., Am
Serviesberg 12, D-5000 Köln 41 (DE)
Erfinder: Bauer, Kurt-Heinz, Prof., Im Finkeler 4,
D-7800 Freiburg 33 (DE)
Erfinder: Wendel, Armin, Goethestrasse 29,
D-5000 Köln 40 (DE)

(74) Vertreter: Sternagel, Hans-Günther, Dr. et al,
Patentanwälte Dr. Michael Hann Dr. H.-G. Sternagel
Sander Aue 30, D-5060 Bergisch Gladbach 2 (DE)

**Beschreibung**

Die Erfindung betrifft neue Wirkstofformulierungen gemäss den Ansprüchen 1–7 und ihre Verwendung zur Herstellung von sogenannten Mikroemulsionen, bei denen die Lipidphase in einer Feinheit von 10–200 nm vorliegt.

Viele aktive Wirkstoffe, wie z.B. pharmazeutische Wirkstoffe oder Pestizide, sind in Wasser schwer oder sogar unlöslich, so dass sich keine homogenen flüssigen Applikationsformen herstellen lassen.

Aus DE-OS 30 42 365 sind flüssige Lecithinhaltige einphasige Mehrstoffsysteme bekannt, die Wirkstoffe enthalten, die in amphiphilen Phospholipiden nicht oder nur wenig löslich sind. Als Viskositätsregler sind Glycerinester von Fettsäuren und/oder acylierte und/oder methoxylierte Derivate dieser Glycerinester oder Polyetherglykole enthalten. Die Systeme sind optisch blanke ölige Flüssigkeiten.

Diese Flüssigkeiten lassen sich durch Einbringen in Wasser jedoch nicht zu stabilen feinteiligen Emulsionen, sogenannten Mikroemulsionen verdünnen, es kommt zu Trennungen, Ausfällungen und inhomogenen Systemen.

Aufgabe der Erfindung ist es, homogene flüssige Wirkstofformulierungen mit in Wasser schwer löslichen oder unlöslichen, aktiven Wirkstoffen oder Wirkstoffmischungen zu schaffen, die sich in jedem Mengenverhältnis mit Wasser verdünnen lassen und dabei als Applikationszubereitungen homogene, durchscheinende Emulsionen bilden.

Diese Aufgabe wird gelöst durch flüssige Wirkstofformulierungen, die einen wasserunlöslichen oder in Wasser schwer löslichen Wirkstoff oder eine Wirkstoffmischung und übliche Zusätze und Hilfsstoffe in einer lipophilen Flüssigkeit oder einem Flüssigkeitsgemisch aus

a) einer Phospholipidmischung aus Phosphatidylcholin, Phosphatidylethanolamin, Phosphatidsäure, Phosphatidylinsitol und/oder N-Acylphosphatidylethanolamin und

b) einem ethoxylierten Derivat eines Glycerinesters mit Fettsäuren als Co-Emulgator, enthalten, die dadurch gekennzeichnet sind, dass ein oder zwei OH-Gruppen des Glycerins des Glycerinesters durch Fettsäuren mit Kohlenwasserstoffketten von 8 bis 16, vorzugsweise 8 bis 10 C-Atomen verestert sind und die 3. bzw. restliche OH-Gruppe des Glycerin-Grundkörpers mit Polyethylenglykol verethert sind und zwar mit 6 bis 30, vorzugsweise 6 bis 15 Ethylenoxid-Einheiten, wobei das molare Verhältnis von Phospholipid zu Co-Emulgator 1:1 bis 1:4 beträgt und die lipophile Flüssigkeit zusätzlich noch Gemische aus Glyceriden, Fettsäuren, niederen Estern, Fettsäureestern und organischen Lösungsmitteln enthält.

Es wurde nun überraschenderweise gefunden, dass man homogene flüssige, transparente Wirkstoffkonzentrate mit wasserunlöslichen oder schwer löslichen Wirkstoffen herstellen kann, die in jedem Verhältnis mit Wasser mischbar sind.

Die erfindungsgemässe flüssige Wirkstofformulierung ist eine lipophile Flüssigkeit, die Phospholipide oder Phospholipidgemische, einen Co-Emulgator und noch lipophile flüssige Bestandteile und den Wirkstoff enthält. Zu diesem lipophilen Wirkstoffkonzentrat in flüssiger Form kann in jedem Mengenverhältnis Wasser zugesetzt werden, ohne dass die optische Transparenz verlorengeht. Hierbei entstehen also Applikationszubereitungen, welche den oder die Wirkstoffe homogen in einer Lipidphase enthalten, die wiederum in dieser applikationsfertigen Zubereitung in O/W-Mikroemulsionsform in Partikelgrössen bzw. Mikrotröpfchen von 10–200 nm vorliegt. Bei diesen durchscheinenden Emulsionen bzw. den Applikationszubereitungen im Sinne dieser Erfindung, die beim Verdünnen der lipophilen Konzentrate mit Wasser entstehen, kann es sich sowohl um mizellare Dispersionen als auch um Mikroemulsionen handeln, bei denen die Tröpfchengrössen der dispersen Phase in kolloiden Grössenordnungen liegen (Leon M. Prince, Microemulsions, Academic Press, Inc.).

Als Phospholipide für die erfindungsgemässen Konzentrat-Formulierungen wird ein Gemisch aus Phosphatidylcholin, Phosphatidylethanolamin, N-Acylphosphatidylethanolamin, Phosphatidsäure oder Phosphatidylinosit verwendet, insbesondere eine Mischung von Phospholipiden mit einem Phosphatidylcholin-Anteil von 30–95%.

Als Co-Emulgator wird Glycerin, bei dem 1 oder 2 OH-Gruppen durch gesättigte Fettsäuren mit Kohlenwasserstoffketten von 8–16, vorzugsweise 8–10 C-Atomen, verestert sind und die dritte oder restliche OH-Gruppe des Glycerin-Grundkörpers mit Polyethylenglykol und zwar mit 6–30, vorzugsweise mit 6–15 Ethylenoxid-Einheiten, verethert ist, zur Anwendung. Die HLB-Werte dieser Co-Emulgatoren (nach Griffin) liegen zwischen 12 und 18, vorzugsweise zwischen 15 und 17.

Beispiele für solche Co-Emulgatoren sind ein wasserlöslich eingestelltes Partialglyceridgemisch von natürlichen gesättigten Pflanzenfettsäuren mittlerer Kettenlänge in Form eines viskosen Öles, umgeesterte simultan polyethoxylierte Caprylsäure/Caprinsäureglyceride (PEG -6- Caprylsäure/Caprinsäureglyceride, Polyoxyethylen-Glycerinmonolaurat mit HLB 15.7, Polyoxyethylen-Glycerinricinocteat mit HLB 14).

Die geeigneten weiteren flüssigen Bestandteile sind mit Phospholipiden verträgliche Glyceride, denen noch geeignete niedere Ester, insbesondere Fettsäureester, beispielsweise Essigsäureethylester, Isopropylmyristat oder Isopropylpalmitat zugesetzt sind.

Um die Verträglichkeit mit Phospholipiden zu verbessern, können ausserdem noch organische Lösungsmittel, die in jedem Verhältnis mit Wasser mischbar sein müssen, hinzugefügt werden, wie beispielsweise niedere Alkohole mit $C_{1-4}$-Kohlenstoffatomen, Dimethylformamid, Dimethylamin oder 2-Dimethyl -4- hydroxymethyl-1,3-dioxalan.

Als weitere Zusätze können übliche Hilfsstoffe, wie Antioxydantien, Komplexbildner oder Konservierungsstoffe zugesetzt werden.

Wesentlich für das Entstehen der Mikroemulsionen als Applikationsform aus den Konzentraten ist das molare Verhältnis von Phospholipid : Co-Emulgator. Dieses kann 1:1 bis 1:4, vorzugsweise 1:1 bis 1:2 betragen. Ganz besonders bevorzugt ist ein Verhältnis von 1:2. Auch der Anteil an Trägerflüssigkeit beeinflusst die Bildung der Mikroemulsion. Der Anteil sollte zwischen 10 und 60 Gew.-% bezogen auf Gesamtmenge Konzentrat-Formulierung betragen.

Die erfindungsgemässen Wirkstoff-Konzentrate, aus denen bei Bedarf durch Zusatz bestimmter Wassermengen die Applikationszubereitungen hergestellt werden, haben folgende Zusammensetzungen:
5–50 Gew.-% Phospholipide
5–80 Gew.-% Co-Emulgator
10–60 Gew.-% Trägerflüssigkeit
0,1–50 Gew.-% Wirkstoff
0,1–10 Gew.-% sonstige Zusätze und Hilfsstoffe.

Zur Herstellung der neuen Wirkstoffkonzentrate wird das Phospholipid in den weiteren flüssigen Bestandteilen unter Rühren und leichter Erwärmung gelöst. In die homogene Flüssigkeit werden der Co-Emulgator und die sonstigen Hilfsstoffe eingerührt. Anschliessend wird der Wirkstoff unter Rühren und leichter Erwärmung bis maximal 60 °C eingebracht und solange weitergerührt, bis eine homogene Phase entsteht. Aus dieser homogenen, vorwiegend lipophilen Phase bildet sich als applikationsfertige Zubereitung eine Mikroemulsion, wenn man sie mit Wasser verdünnt.

Als Wirkstoffe können wasserunlösliche oder schwer lösliche pharmazeutische Wirkstoffe, Pflanzenschutzmittel mit herbizider, fungizider, insektizider, acarizider, nematizider oder Pflanzenwachstum-regulierender Wirkung verwendet werden.

Aus der Gruppe der Herbizide z.B.:
N-Phosphonomethylglycin (Glyphosat),
3-(3-Chlor -4- methyl-phenyl)-1,1-dimethylharnstoff (Chlortoluron),
N-(4-Methoxy -6- methyl-1,3,5-triazin -2-yl)-aminocarbonyl -2- chlorphenylsulfonamid,
3-(4-Isopropyl-phenyl)-1,1-dimethylharnstoff (Isoproturon),
3-Methyl -4- amino -6- phenyl-1,2,4-triazin-5(4H)-on (Metamitron),
1,3-Dimethyl -3- (2-benzthiazolyl)-harnstoff (Methabenzthiazuron),
2-Chlor -4- ethylamino -6- isopropylamino -s-triazin (Atrazin),
3-(3,4-Dichlorphenyl) -1- methoxy -1-methylharnstoff (Linuron),
3,5-Dibrom -4- hydroxybenzaldehyd -0-(2,4-dinitrophenyl)-oxim (Bromfenoxim),
3-[4-(Chlorphenoxy)-phenyl]-1,1-dimethylharnstoff (Chloroxuron),
2,6-Dichlor-thio-benzamid (Chlorthiamid),
N,N-Dimethyl-2,2-diphenylacetamid (Diphenamid),
3-(3,4-Dichlorphenyl)-1,1-dimethylharnstoff (Diuron),

2-(3,4-Dichlorphenyl) -4- methyl-1,2,4-oxa-diazolidin-3,5-dion (Methazol),
3-(p-Chlorphenyl)-1,1-dimethylharnstoff (Monuron),
3-(3,4-Dichlorphenyl) -1- methyl -1- n-butylharnstoff (Neburon),
2-Chlor-4,6-bis-ethylamino-s-triazin (Simazin), oder
3-tert.-Butyl -5- chlor -6- methyluracil (Terbacil);
aus der Gruppe der Fungizide z.B.:
1,3-Dicyan-2,4,5,6-tetrachlorbenzol (Chlorthalonil),
N-Trichlormethylthiophthalimid (Folpet),
N-(Trichlormethylthio)-tetrahydrophthalimid (Captan),
1-(Butylcarbamoyl)-benzimidazol -2-yl-carbamat (Benomyl),
2,4-Dichlor -6- (2-chloranilin)-1,3,5-triazin (Anilazin),
2-(Methoxy-carbonylamino)-benzimidazol (Carbendazim),
6-Methyl -2- oxo-1,3-dithiol [4,5-b]-chinoxalin (Chinomethionat),
Triphenylzinnacetat (Fentin-acetat),
Eisendimethyldithiocarbamat (Ferbam),
N-Trichlormethylthiopthalimid (Folpet),
Kupferoxychlorid,
Mangan-Zink-ethylendiamin-bis-dithio-carbamat (Manozeb),
Mangan-(II)-[N,N'-ethylen-bis(dithiocarbamat)] (Maneb), oder
Tetramethyl-thiuram-disulfid (Thiram);
oder z.B. folgende Insektizide:
2,3-Dihydro-2,2-dimethyl-benzofuran -7-yl-N-methylcarbamat (Carbofuran),
0,S-Dimethyl-N-acetyl-aminothiophosphat (Acephat),
1-(4-Chlorphenyl) -3- (2,6-difluorbenzoyl)-harnstoff (Diflubenzuron),
6-Chlor-3,4-xylyl-N-methylcarbamat (Carbanolat), oder
Endrin.
2-(3,4-Dichlorphenyl) -4- methyl-1,2,4-oxa-diazolidin-3,5-dion (Methazol),
3-(p-Chlorphenyl)-1,1-dimethylharnstoff (Monuron),
3-(3,4-Dichlorphenyl) -1- methyl -1- n-butylharnstoff (Neburon),
2-Chlor-4,6-bis-ethylamino-s-triazin (Simazin), oder
3-tert.-Butyl -5- chlor -6- methyluracil (Terbacil);
aus der Gruppe der Fungizide z.B.:
1,3-Dicyan-2,4,5,6-tetrachlorbenzol (Chlorthalonil),
N-Trichlormethylthiophthalimid (Folpet),
N-(Trichlormethylthio)-tetrahydrophthalimid (Captan),
1-(Butylcarbamoyl)-benzimidazol -2- yl-carbamat (Benomyl),
2,4-Dichlor -6- (2-chloranilin)-1,3,5-triazin (Anilazin),
2-(Methoxy-carbonylamino)-benzimidazol (Carbendazim),

6-Methyl -2- oxo-1,3-dithiol [4,5-b]-chinoxalin (Chinomethionat),

Triphenylzinnacetat (Fentin-acetat),

Eisendimethyldithiocarbamat (Ferbam),

N-Trichlormethylthiopthalimid (Folpet),

Kupferoxychlorid,

Mangan-Zink-ethylendiamin-bis-dithio-carbamat (Manozeb),

Mangan-(II)-[N,N'-ethylen-bis(dithiocarbamat)] (Maneb), oder

Tetramethyl-thiuram-disulfid (Thiram);

oder z.B. folgende Insektizide:

2,3-Dihydro-2,2-dimethyl-benzofuran -7-yl-N-methylcarbamat (Carbofuran),

0,S-Dimethyl-N-acetyl-aminothiophosphat (Acephat),

1-(4-Chlorphenyl) -3- (2,6-difluorbenzoyl)-harnstoff (Diflubenzuron),

6-Chlor-3,4-xylyl-N-methylcarbamat (Carbanolat), oder

Endrin.

Besondere Bedeutung hat die erfindungsgemässe Formulierungsmöglichkeit auch für pharmazeutische Wirkstoffe, weil sich damit injizierbare flüssige Konzentrate herstellen lassen, die nach Verdünnungen mit Wasser p.i. direkt injizierbar sind. Dies war mit wasserunlöslichen oder auch flüssigen, lipophilen Pharmazeutika bisher nicht auf so einfache Weise möglich.

Geeignete pharmazeutische Wirkstoffe sind beispielsweise: Acetylsalizylsäure, Ibuprofen, Benzodiazepine, Nitroglycerin, Isosorbiddinitrat, Corticoide, Chloramphenicol, Dexamethson, Mebendazol, Griseofulvin, Miconazol, Nitrofurantoin, Furosemid, Clotrimazol, Metronidazol.

Die Erfindung wird nun anhand von Beispielen noch näher erläutert:

**Beispiel 1**

| | |
|---|---|
| Isopropylmyristat | 40,0 % |
| Phospholipid | 20,0 % |
| PEG -6- Caprylsäure/Caprinsäure-Glycerid | 21,0 % |
| Na-EDTA | 0,1 % |
| Butylhydroxytoluol (BHT) | 0,02% |
| p-Hydroxybenzoesäuremethylester | 0,15% |
| Ethanol | 13,73% |
| Wirkstoff: Chloramphenicolpalmitat | 5,0 % |

Das Phospholipid wird in Ethanol und Softigen durch Rühren gelöst. Dazu wird Isopropylmyristat, Na-EDTA, Butylhydroxytoluol und p-Hydroxybenzoesäuremethylester gegeben und solange gerührt, bis eine homogene Phase entsteht, dazu wird unter Rühren und Erwärmen auf 50 °C Chloramphenicolpalmitat gegeben. Es entsteht ein Wirkstoffkonzentrat, welches beim Verdünnen mit Wasser ein optisch transparentes, homogenes System in Form einer Mikroemulsion bildet.

Analog Beispiel 1 werden die folgenden Beispiele hergestellt:

**Beispiel 2**

| | |
|---|---|
| Caprylsäure/Caprinsäureglycerid | 25,0 kg |
| Phospholipid | 31,05 kg |
| PEG -6- Caprylsäure/Caprinsäureglycerid | 35,0 kg |
| Ascorbylpalmitat | 0,01 kg |
| alpha-Tocopherol | 0,05 kg |
| Methylhydroxybenzoat | 0,2 kg |
| Propylhydroxybenzoat | 0,1 kg |
| 2,2-Dimethyl -4- hydroxymethyl-1,3-dioxalan | 3,64 kg |
| Wirkstoff: Chlordiazon | 5,0 kg |

**Beispiel 3**

| | |
|---|---|
| Essigsäureethylester | 32,0 g |
| Phospholipid | 31,0 g |
| umgeesterte simultan polyethoxylierte Caprylsäure/Caprinsäureglyceride | 33,95 g |
| alpha-Tocopherol | 0,05 g |
| Wirkstoff: Ektrimfos | 1,0 g |

**Beispiel 4**

| | |
|---|---|
| Glycerintriacetat | 15,0 g |
| Phospholipid | 17,5 g |
| Polyoxyethylen-Glycerinmonolaurat mit HLB 15.7 | 17,5 g |
| Ascorbylmyristat | 0,01 g |
| Na-EDTA | 0,2 g |
| Ethanol | 37,99 g |
| Wirkstoff: Triadimefon | 10,0 g |

**Beispiel 5**

(Miglyol 812)

| | |
|---|---|
| Caprylsäure-Caprinsäure-Triglycerid mit einem Molgewicht 520 | 22,0 g |
| Phospholipid | 30,0 g |
| Polyoxyethylen-Glycerinricinocteat | 29,0 g |
| Na-EDTA | 0,1 g |
| Butylhydroxyanisol (BHA) | 0,01 g |
| Isopropanol | 13,89 g |
| Wirkstoff: Dexamethasonacetat | 5,0 g |

Die erfindungsgemässen Wirkstofformulierungen lassen sich in analoger Weise wie bekannte Wirkstofformulierungen verwenden, haben jedoch den Vorzug, dass sie beim Verdünnen mit Wasser in makroskopischen Sinne homogene bzw. kolloidale Systeme ergeben, also praktisch nicht sedimentierende oder aggregierende Applikationszubereitungen im Gegensatz zu den bisher bekannten inhomogenen flüssigen, grob dispersen Systemen sind. Dadurch ist die Palette der Einsatzmöglichkeiten erheblich erweitert.

**Patentansprüche**

1. Flüssige Wirkstofformulierungen, die einen wasserunlöslichen oder in Wasser schwer löslichen Wirkstoff oder eine Wirkstoffmischung und übliche Zusätze und Hilfsstoffe in einer lipophilen Flüssigkeit oder einem Flüssigkeitsgemisch aus

a) einer Phospholipidmischung aus Phosphatidylcholin, Phosphatidylethanolamin, Phosphatidsäure, Phosphatidylinsitol und/oder N-Acylphosphatidylethanolamin und

b) einem ethoxylierten Derivat eines Glycerinesters mit Fettsäuren als Co-Emulgator, enthalten, dadurch gekennzeichnet, dass ein oder zwei OH-Gruppen des Glycerins des Glycerinesters durch Fettsäuren mit Kohlenwasserstoffketten von 8 bis 16, vorzugsweise 8 bis 10 C-Atomen verestert sind und die 3. bzw. restliche OH-Gruppe des Glycerin-Grundkörpers mit Polyethylenglykol verethert sind und zwar mit 6 bis 30, vorzugsweise 6 bis 15 Ethylenoxid-Einheiten, wobei das molare Verhältnis von Phospholipid zu Co-Emulgator 1:1 bis 1:4 beträgt und die lipophile Flüssigkeit zusätzlich noch Gemische aus Glyceriden, Fettsäuren, niederen Estern, Fettsäureestern und organischen Lösungsmitteln enthält.

2. Wirkstofformulierungen nach Anspruch 1, dadurch gekennzeichnet, dass das molare Verhältnis von Phospholipid zu Co-Emulgator 1:1 bis 1:2 beträgt.

3. Wirkstofformulierungen nach Anspruch 1, dadurch gekennzeichnet, dass das molare Verhältnis von Phospholipid zu Co-Emulgator 1:2 beträgt.

4. Wirkstofformulierungen nach Ansprüchen 1 bis 4, dadurch gekennzeichnet, dass sie aus

5 bis 50 Gew.-% Phospholipiden

5 bis 80 Gew.-% Co-Emulgator

10 bis 60 Gew.-% Gemisch aus Glyceriden, Fettsäuren und organischen Lösungsmitteln

0,1 bis 50 Gew.-% Wirkstoff oder Wirkstoffgemisch und

0,1 bis 10 Gew.-% sonstigen Zusätzen und Hilfsstoffen bestehen.

5. Wirkstofformulierungen nach einem der vorangehenden Ansprüche, dadurch gekennzeichnet, dass sie als Wirkstoff oder Wirkstoffgemische einen biologisch aktiven Stoff oder eine Mischung davon enthalten.

6. Wirkstofformulierungen nach einem der vorangehenden Ansprüche, dadurch gekennzeichnet, dass sie als Wirkstoff ein in Wasser unlösliches oder schwer lösliches Pestizid enthalten.

7. Wirkstofformulierungen nach einem der Ansprüche 1 bis 5, dadurch gekennzeichnet, dass sie als Wirkstoff Acetylsalizylsäure, Ibuprofen, Benzodiazepine, Nitroglycerin, Isosorbiddinitrat, Corticoide, Choramphenicol, Dexamethson, Mebendazol, Griseofulvin, Miconazol, Nitrofurantoin, Furosemid, Clotrimazol, Metronidazol enthalten.

8. Verwendung der Wirkstofformulierungen nach den Ansprüchen 1 bis 7 zur Herstellung optisch transparenter wässriger Flüssigkeitssysteme, in denen die Lipidphase mit dem Wirkstoff in einer Feinheit von 10–200 nm vorliegt.

**Revendications**

1. Formules de substances actives liquides, qui contiennent une substance active ou un mélange de substances actives insolubles dans l'eau ou peu solubles dans l'eau et des additifs et adjuvants habituels dans un liquide ou un mélange de liquides lipophiles constitué:

a) d'un mélange de phospholipides constitués de phosphatidylcholine, de phosphatidyléthanolamine, d'acide phosphatidique, de phosphatidylinositol et/ou de N-acylphosphatidyléthanolamine et

b) d'un dérivé éthoxylé d'un ester du glycérol, avec des acides gras comme co-émulsionnants, caractérisées par le fait qu'un ou deux groupes OH du glycérol ou de l'ester du glycérol sont estérifiés par des acides gras ayant des chaînes hydrocarbonées de 8 à 16, de préférence de 8 à 10 atomes de carbone, et le troisième groupe OH ou groupe OH restant de la substance de base glycérol est éthérifié avec du polyéthylène glycol, et ceci avec 6 à 30, de préférence 6 à 15 motifs oxyde d'éthylène, le rapport molaire du phospholipide au co-émulsionnant étant de 1:1 à 1:4, et le liquide lipophile contenant en outre des mélanges de glycérides, d'acides gras, et d'esters inférieurs, d'esters d'acides gras et de solvants organiques.

2. Formules de substances actives selon la revendication 1, caractérisées par le fait que le rapport molaire du phospholipide au co-émulsionnant est de 1:1 à 1:2

3. Formules de substances actives selon la revendication 1, caractérisées par le fait que le rapport molaire du phospholipide au co-émulsionnant est de 1:2.

4. Formules de substances actives selon les revendications 1 à 4, caractérisées par le fait qu'elles se composent de

5 à 50% en poids de phospholipides

5 à 80% en poids de co-émulsionnant

10 à 60% en poids d'un mélange de glycérides, d'acides gras et de solvants organiques

0,1 à 50% en poids de substance active ou d'un mélange de substances actives, et

0,1 à 10% en poids d'autres additifs et adjuvants.

5. Formules de substances actives selon l'une des revendications précédentes, caractérisées par le fait qu'elles contiennent comme substance active ou comme mélanges de substances actives une substance ou un mélange de substances biologiquement actives.

6. Formules de substances actives selon l'une des revendications précédentes, caractérisées par le fait qu'elles contiennent comme substance active un pesticide insoluble ou peu soluble dans l'eau.

7. Formules de substances actives selon l'une des revendications 1 à 5, caractérisées par le fait qu'elles contiennent comme substances actives de l'acide salicylique, de l'ibuprofène, de la benzodiazépine, de la nitroglycérine, du dinitrate d'isosorbide, des corticoïdes, du chloramphénicol, de la dexaméthazone, du mébendazole, de la griséofulvine, du miconazole, de la nitrofurantoine, du furosémide, du clotrimazole, du métronidazole.

8. Utilisation des formules de substances actives selon les revendications 1 à 7 pour la préparation de systèmes de liquides aqueux optiquement transparents, dans lesquels la phase lipidique est présente dans une finesse de 10–200 nm.

**Claims**

1. Liquid active material formulations which contain an active material which is water-insoluble or difficultly soluble in water or an active material mixture and conventional additives and auxiliaries in a lipophil liquid or a liquid mixture of

a) a phospholipid mixture of phosphatidyl choline, phosphatidyl ethanolamine, phosphatide acid, phosphatidyl insitol and/or N-acylphosphatidyl ethanolamine and

b) an ethoxylated derivative of a glycerin ester with fatty acids as co-emulsifier, characterised in that, one or two OH groups of the glycerin of the glycerin ester are esterified by fatty acids with hydrocarbon chains having from 8 to 16, preferably from 8 to 10 carbon atoms and the 3 or remaining OH groups of the glycerin parent substance are esterified with polyethylene glycol, with from 6 to 30, preferably from 6 to 15 ethylene oxide units, whereby the molar ratio of phospholipid to co-emulsifier is from 1:1 to 1:4 and the lipophilic liquid additionally contains mixtures of glycerides, fatty acids, lower esters, fatty acid esters and organic solvents.

2. Active material formulations according to claim 1, characterised in that, the molar ratio of phospholipid to co-emulsifier is from 1:1 to 1:2.

3. Active material formulations according to claim 1, characterised in that, the molar ratio of phospholipid to co-emulsifier is 1:2.

4. Active material formulations according to any of Claims 1 to 4, characterised in that, they consist of

from 5 to 50% by weight of phospholipids
from 5 to 80% by weight of co-emulsifier
from 10 to 60% by weight of a mixture of glycerides, fatty acids and organic solvents,
from 0,1 to 50% by weight of active material or a mixture of active materials
from 0,1 to 10% by weight of other additives and auxiliaries.

5. Active material formulations according to one of the preceding claims, characterised in that, they contain a biologically active material or a mixture thereof as the active material or mixtures of active materials.

6. Active material formulations according to one of the preceding claims, characterised in that, they contain a pesticide which is insoluble or difficultly soluble in water as the active material.

7. Active material formulations according to one of claims 1 to 5, characterised in that, they contain as the active material, acetylsalicylic acid ibuprofen, benzodiazepines, nitroglycerin, isosorbide dinitrate, corticoids, chloramphenicol, dexamethasone, mebendazole, griseofulvin, miconazole, nitrofurantoin, furosemide, clotrimazole, metronidazole.

8. Use of the active material formulations according to any of Claims 1 to 7 for the production of optically transparent aqueous liquid systems, in which the lipid phase with the active material is present in a fineness of from 10 to 200 nm.